# EUROPEAN PATENT SPECIFICATION

(11) **EP 1 782 815 B1**
(45) Date of publication and mention of the grant of the patent: **08.09.2010**
(21) Application number: 05764495.7
(22) Date of filing: 23.06.2005
(51) Int. Cl.: A61K 31/64, A61P 25/00

(54) **COMPOUND FOR USE IN THE DIAGNOSIS OF CNS ACUTE DAMAGE**
ZUSAMMENSETZUNG ZUR VERWENDUNG BEI DIAGNOSE DER AKUTEN VERLETZUNGEN DES ZENTRALEN NERVENSYSTEMS
COMPOSE DESTINE A ETRE UTILISE DANS LE DIAGNOSTIC D'UNE LESION AIGUE DU SYSTEME NERVEUX CENTRAL

(30) Priority: 23.06.2004 ES 200401628
(43) Date of publication of application: 09.05.2007
(73) Proprietor: Neurotec Pharma, S.L., 08028 Barcelona (ES)
(72) Inventor: MAHY GÉHENNE, Josette-Nicole, E-08328 Alella (ES); RODRÍGUEZ ALLUÉ , Manuel José, E-08980 Sant Feliu de Llobregat (ES); PUGLIESE, Marco, E-08810 Sant Pere de Ribes (ES)
(74) Representative: ABG Patentes, S.L.
(86) International application number: PCT/ES2005/000357
(87) International publication number: WO 2006/000608

(56) References cited:
- WO-A1-2004/093896
- US-A- 6 147 087
- BINGHAM E. ET AL: 'The effects of KATP channel modulators on counterregulatory responses and cognitive function during acute controlled hypoglycaemia in healthy men: a pilot study' DIABETIC MEDICINE vol. 20, no. 3, 2003, pages 231 - 237, XP003009243
- STEIN J.: 'Fampridine may help patients with spinal cord injury:', [Online] 15 October 2002, XP003009244 Retrieved from the Internet: <URL:http://www.pslgroup.com/dg/22068A.htm>

## Description

This invention relates to an isotopically modified KATP channel closer (kcc) for use in the diagnosis of CNS acute damage in mammal, including a human, wherein the damage is caused by a CNS injury.

### BACKGROUND ART

Microglia are distributed in non-overlapping territories throughout the Central Nervous System (CNS). In functional terms, microglia represents the network of immune accessory cells throughout the brain, spinal cord and eye neurostructures functioning as an intrinsic sensor of threats. The high sensitivity of microglial cells to the CNS microenvironment changes enables them to function as sentinels (cf. G.W. Kreutzberg, Trends Neurosci. 1996, vol. 19, pp. 312-8). Benefits derived from activated microglia remain controversial because of its dual role, protecting the CNS from damage as well as amplifying the effects of inflammation and autoimmune responses and mediating cellular neurodegeneration (cf. W.J. Streit et al., Prog. Neurobiol. 1999, vol. 57, pp. 563-81).

CNS damage rapidly changes neuronal gene expression and stimulates nearby microglia for support. Microglia activation, the first step in the protection of CNS injury (cf. L. Minghetti et al., Prog. Neurobiol. 1998, vol. 54, pp. 99-125) is sufficient to restrain further tissue damage. After an injury, early activated microglial cells secrete anti-inflammatory cytokines (e.g. IL-10 and TGF-beta) and express glutamate transporters to prevent excitotoxic injury.

Thus, in this acute phase, the glutamate clearance by active astrocyte is helped by a de novo microglial Glu transporter expression (cf. A.V. Vallat-Decouvelaere et al., J. Neuropathol. Exp. Neurol. 2003, vol. 62, pp. 475-85) to avoid excitotoxic damage. In this early post-injury event, ramified microglial cell expression of EAAT1, EAAT2 and EAAT3 glutamate transporters prevents glutamate-mediated excitatory neuronal cell death (cf. F. Lopez-Redondo et al., Brain Res. Mol. Brain Res. 2000, vol. 76, pp. 429-35; F. 35 Chretien etal., Neuropathol. Appl. Neurobiol. 2002, vol. 28, pp. 410-7).

At present, very few treatments have been proposed in clinical practice to prevent CNS acute damage. They are orientated to inhibit or prevent activation of mechanisms involved in neuronal death, but their effectiveness is limited and sometimes contradictory. Some agents, such as tirilazad mesylate and Ebselen (2-phenyl-1,2-benzisoselenazol-3(2H)-one) have been proposed to neutralize free radicals and avoid their toxicity; others have been proposed to reduce intracellular calcium toxicity (e.g. nimodipine) or to interfere with GABAergic neurotransmission (e.g. clomethiazol) or with glutamate neurotransmission (e.g. magnesium). However, at present, there is not any treatment to avoid CNS acute injury presenting both a good efficacy and a high security for the patient.

Actually, in most of the times, after an acute damage, patients are kept under clinical observation during some days in absence of a specific treatment just waiting for a beneficial evolution. Thus, it is desirable to provide new therapeutic agents for the early treatment of CNS acute damage.

### SUMMARY OF THE INVENTION

Inventors have surprisingly found that human and rodent activated microglia strongly expresses a K_{ATP} channel similar to the ones known in cardiac and muscular tissues, neurones and pancreatic beta cells. K_{ATP} channels initially found in heart (cf. A. Noma, Nature 1983, vol. 305, pp. 147-8) have also been described in pancreas, skeletal muscle, smooth muscle, pituitary, tubular cells of the kidney, vascular cells and specific neurons of some brain areas.

The fact that activated microglia expresses K_{ATP} channels, turns K_{ATP} channel closers (KCCs) including sulfonylureas, into therapeutic targets to protect CNS from acute damage. KCCs have been used until now for the treatment of diabetes type 2. Inventors have found that the KCCs, and in particular glibenclamide, potentiate acute microglial reaction and avoid AMPA-induced (AMPA: α-amino-3-hydroxy-5-methylisoxazole-4-propionic) brain excitotoxicity in various CNS pathologies such as stroke, seizure, axonal injury, traumatic damage, neurodegeneration, spinal cord injury, infectious and autoimmune diseases. KCCs promote synaptic glutamate removal and anti-inflammatory cytokine secretion by ramified microglia at early survival periods.

Thus, the present invention relates to the use of an isotopically modified KCC, for use in the diagnosis of CNS acute damage in a mammal, including a human, and wherein said damage involves the activation of microglia expressing KATP channels wherein said use comprises the administration of an effective amount of an isotopically modified KCC, together with appropriate amounts of acceptable diluents or carriers.

KCCs are typically sulfonylureas. Examples of them are glibenclamide, tolbutamide, gliclazide, gliquidone, tolazamide, chlorpropamide, glipizide, glyburide, glimepiride and glisentide. In a particular embodiment of the invention, the KCC is glibenclamide.

In a particular embodiment of the invention, the CNS acute damage is caused by a CNS injury, such as brain injury, spinal cord injury, global ischemia, focal ischemia, hypoxia, stroke, seizure, epilepsy, status epilepticus, CNS vascular disease, neuroocular disease (e.g. inflammation optic neuropathy and retinitis) and trauma.

According to the present invention, KCCs prevent CNS acute excitotoxic effects.

A person skilled in the art would select an appropriate administration via of KCCs, including glibenclamide, such as oral, buccal, parenteral, depot or rectal administration, or by inhalation or insufflation (either through the mouth or the nose). Oral and parenteral formulations are preferred. Their administration is preferred to be associated with a narrow therapeutic window following the acute damage.

For oral administration, KCCs may take the form of, for example, tablets or capsules prepared by conventional means with pharmaceutically acceptable excipients. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives. Preparations for oral administration may be suitably formulated to give controlled release of the active compound.

Liquid preparations for perioperative CNS surgery including brain, spinal cord and neuroophthalmic procedures may take the form of, for example, solutions or suspensions, or they may be presented as a dry product for its direct application (e.g. powder, gel or impregnated on a solid support) or reconstitution with water or other suitable vehicle (e.g. sterile pyrogen-free water) before use. Such liquid preparations may be prepared by conventional means with pharmaceutically acceptable additives such as emulsifying agents (e.g. lecithin or acacia); nonaqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, and, optionally, multiple active agents (e.g. antibiotics) in a physiological carrier, such as saline or lactated Ringer's solution, as appropriate. The solution is applied by continuous irrigation of a wound during surgical and diagnostic procedures to potentiate neuroprotection of the CNS.

KCCs may be formulated for parental administration by bolus injection or continuous infusion. Formulations for injection may be presented in unit dosage form (e.g. in ampoules or in multidose containers) with an added preservative. The compositions may take forms such as suspensions, solutions or emulsions in oily or aqueous vehicles, and may contain agents such as stabilizing and/or dispersing agents. Alternatively, the active ingredient may be in powder form for constitution with a suitable vehicle (e.g. sterile pyrogen-free water) before use.

KCCs may also be formulated for local administration, for example, by carotid injection, lumbar or cisternal puncture, intracerebroventricular or tissue infusion, as solutions for administration via a suitable delivery device or alternatively as a powder mix with a suitable carrier for administration using a suitable delivery device.

KCCs may also be formulated as rectal compositions such as suppositories or retention enemas (e.g. containing conventional suppository bases such as cocoa buffer or other glycerides).

For intranasal and ocular administration, KCCs may be formulated as solutions for administration via a suitable metered or unit dose device or alternatively as a powder mix with a suitable carrier for administration using a suitable delivery device.

Suitable doses ranges would be routinely found by the person skilled in the art. Thus, for use in conditions according to the present invention, the compounds may be used at doses appropriate for other conditions for which KCCs are known to be useful. It will be appreciated that it may be necessary to make routine variations to the dosage, depending on the age and condition of the patient, and the precise dosage will be ultimately at the discretion of the attendant physician or veterinarian. The dosage will also depend on the route of administration and the particular compound selected. A suitable dose range is for example 0.01 to 1000 mg/kg bodyweight per day, preferably from 0.1 to about 200 mg/kg and more preferably from 0.1 mg/kg to 10 mg/kg,.

The KCCs useful in the present invention may be administered in combination with other KCCs and/or in combination with other therapeutic agents and may be formulated for administration by any convenient route in a convenient manner. Appropriate doses would be routinely found by those skilled in the art.

The invention refers to the use of an isotopically modified KCC for the preparation of a diagnostic agent for CNS acute damage. The skilled in the art would appropiately choose isotopes and techniques to detect and follow microglial reaction. Functional brain imaging techniques such as positron emission tomography (PET), single-photon emission computed tomography (SPECT) and nuclear magnetic resonance (NMR) may provide an image that represents the distribution in the CNS of the microglial reaction. Once activated, microglia shows a territorially highly restricted involvement in the disease process. This confers to them diagnostic value for the accurate spatial localization of any active disease process. KCCs may be labelled for example with ¹¹C, ¹³C, ¹⁷F, ³¹P, ¹H or ¹⁷O.

### BRIEF DESCRIPTION OF THE DRAWINGS

FIG. 1 shows the hippocampal microgliosis area (A, in mm²) induced by stereotaxic microinjection of PBS (sham, S), glibenclamide (Glib), AMPA and AMPA+glibenclamide (AMPA+Glib). One asterisk means p< 0.01 different from sham and the symbol # means p< 0.01 different from AMPA (LSD post-hoc test).
FIG 2. shows the area (A, in mm²) of hippocampal CA1 lesion induced by stereotaxic microinjection of PBS (sham, S), glibenclamide (Glib), AMPA, or AMPA+glibenclamide (AMPA+Glib). One asterisk means p< 0.01 different 35 from sham, and the symbol # means p< 0.01 different from AMPA (LSD post-hoc test).

EXAMPLES: The present invention is based on the discovery that Glibenclamide potentiates microglial reaction and avoids AMPA induced rat hippocampal excitotoxic damage. The example demonstrating said effect is provided:

This model relies on the acute stereotaxic over-activation of rat glutamate hippocampal receptors that results in a neurodegenerative process characterized by a neuronal loss with astroglial and microglial reactions (cf. F. Bernal et al., Hippocampus 2000, vol. 10, pp. 296-304; F. Bernal et al., Exp. Neurol. 2000, vol. 161, pp. 686-95). In this neurodegenerative model, rats were anaesthetized with equithesin (a mixture of chloral hydrate and sodium pentobarbitone; 0.3 ml/100 g body wt, i.p.), and placed on a Kopf stereotaxic frame with the incisor bar set at -3.3 mm. Intracerebral injections aimed at the dorsal hippocampus were performed at 3.3 mm caudal to bregma, 2.2 mm lateral, and 2.9 mm ventral from dura (cf. G. Paxinos et al., "The rat brain in stereotaxic coordinates", Sydney: Academic Press 1986). A volume of 0.5 µ*l* was injected over a period of 5 min.

Four different groups of rats received two injections in a 2-hour interval as follows: a) sham rats (n = 4) received two injections of PBS; b) AMPA rats (n = 4) received the first injection of 5.4 mM AMPA and the second of PBS; c) glibenclamide rats (n = 4) received two injections of 20 µ*M* glibenclamide; d) AMPA+glibenclamide rats (n = 4) received 5.4 mM AMPA + 20 µ*M* glibenclamide in the first injection and 20 µ*M* glibenclamide in the second injection. All rats were sacrified 24 hours after the lesion.

Rats were transcardially perfused with 300 ml of 0.1 M phosphate buffer (PB, pH 7.4) followed by 300 ml ice-cold fixative (flow rate 20 ml/min). The fixative consisted of 4% (w/v) paraformaldehyde in PB. Brains were removed, crioprotected with 15% (w/v) sucrose in PB and then, frozen with dry ice.

Cryostat sections (12 µ*m)* were obtained at the level of dorsal hippocampus (-3.3 mm to bregma). Isolectine B4 (IB4) histochemistry was performed to identify the microglial reaction (cf. C.A. Colton et al., J. Histochem. Cytochem. 1992, vol. 40, pp. 505-12). The hippocampal morphology was studied in Cresyl violet stained sections. The area of lesion and the microgliosis evaluation were performed on cresyl violet and IB4-positive stained sections respectively. These parameters were analyzed using a computer-assisted image analysis system (OPTIMAS®, BioScan Inc., Washington, USA). IB4-stained reactive microcytes were counted at x100 magnification using an ocular grid mounted on a transmission light microscope (Axiolab, Zeiss, Göttingen, Germany). One-way ANOVA was used to compare differences between groups, followed by the LSD post-hoc test. Results are expressed as mean ± SEM. All analyses were performed with the computer program STATGRAPHICS (STSC Inc., Rockville, MD, USA).

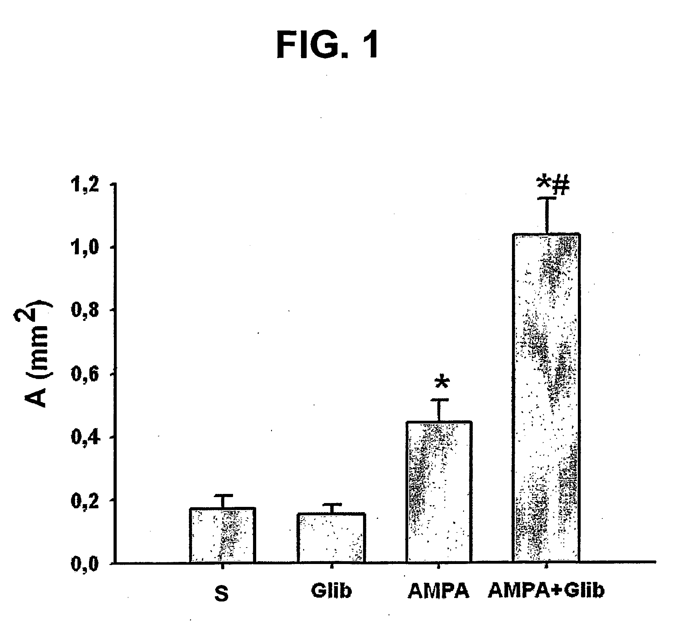

The microglial reaction found in sham and glibenclamide groups was similar, reaching an area of 0.17 ± 0.04 mm² and 0.16 ± 0.03 mm² respectively. In AMPA rats, a strong microgliosis was evidenced with the ameboid microcytes extended through an area of 0.44 ± 0.07 mm². In the AMPA+Glib group this microgliosis area was increased to an area of 1.04 ± 0.11 mm² (236% of the AMPA group) (One-way ANOVA test result: F₃,1₂=31.81; p=0.0001) (cf. FIG. 1).

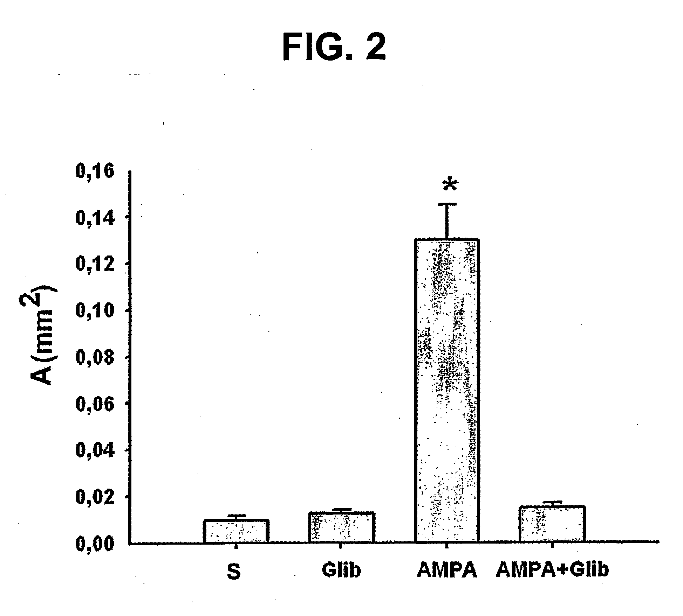

In all four groups the density of reactive microcytes found was similar: 504 ± 82 cells/mm² for sham, 614 ± 91 cells/mm² for glibenclamide, 645 ± 59 cells/mm² for AMPA and 568 ± 56 cells/mm² for AMPA+Glib. As illustrated in FIG. 2 with the quantification of the CA1 pyramidal layer, rich in neuronal cells, in this last group, the 236% increased microglial reaction was associated with an absence of a significant hippocampal lesion. In this layer, the 0.130 ± 0.015 mm² of lesion observed in AMPA rats were decreased to 0.015 ± 0.0016 mm² in the AMPA+Glib group, similar to the areas of 0.009 ± 0.0015 mm² and 0.012 ± 0.0017 mm² found in the sham and glibenclamide groups respectively (One-way ANOVA test result: F₃,11=52.14; p=0.00001).

From these results it is clear that glibenclamide potentiates microglial activation and avoids hippocampal excitotoxic damage. A lack of hippocampal lesion was observed in animals treated with AMPA+glibenclamide in comparison with AMPA treated animals.

## Claims

1. An isotopically modified K_{ATP} channel closer (KCC), for use in the diagnosis of CNS acute damage in a mammal, including a human, wherein the damage is caused by a CNS injury.

2. An isotopically modified K_{ATP} channel closer for use according to claim 1, wherein the K_{ATP} channel closer is a sulfonylurea.

3. An isotopically modified K_{ATP} channel closer for use according to any preceding claim wherein the isotopically modified K_{ATP} channel closer (KCC) is labelled with ¹¹C, ¹³C, ¹⁷F, ³¹P, ¹H or ¹⁷O.

4. An isotopically modified K_{ATP} channel closer for use according to any preceding claim, wherein the CNS injury is selected from the group consisting of brain injury, spinal cord injury, global ischemia, focal ischemia, hypoxia, stroke, seizure, epilepsy, status epilepticus, CNS vascular disease, neuroocular disease and trauma.

5. An isotopically modified K_{ATP} channel closer for use according to any of the preceding claims, wherein the K_{ATP} channel closer is glibenclamide.

## Patentansprüche

1. Isotopisch modifizierter K_{ATP}-Kanalabschluss (KCC) zur Verwendung in der Diagnose einer akuten Schädigung des zentralen Nervensystems bei einem Säugetier, einschließend einen Menschen, wobei die Schädigung durch eine Verletzung des zentralen Nervensystems verursacht wird.

2. Isotopisch modifizierter K_{ATP}-Kanalabschluss zur Verwendung nach Anspruch 1, wobei der K_{ATP}-Kanalabschluss ein Sulfonylharnstoff ist.

3. Isotopisch modifizierter K_{ATP}-Kanalabschluss zur Verwendung gemäß einem vorangehenden Anspruch, wobei der isotopisch modifizierte K_{ATP}-Kanalabschluss (KCC) mit ¹¹C, ¹³C, ¹⁷F, ³¹P, ¹H oder ¹⁷O markiert ist.

4. Isotopisch modifizierter K_{ATP}-Kanalabschluss zur Verwendung nach einem vorangehenden Anspruch, wobei die Verletzung des zentralen Nervensystems ausgewählt ist aus der Gruppe bestehend aus einer Gehirnverletzung, einer Rückenmarksverletzung, einer globalen Ischämie, einer fokalen Ischämie, Hypoxie, Schlaganfall, Epilepsie, Status Epilepticus, vaskulärer CNS-Erkrankung, neurookularer Erkrankung und Trauma.

5. Isotopisch modifizierter K_{ATP}-Kanalabschluss zur Verwendung nach einem der vorangehenden Ansprüche, wobei der K_{ATP}-Kanalabschluss Glibenclamid ist.

## Revendications

1. Bloqueur de canaux K_{ATP} (KCC) modifié isotopiquement pour être utilisé dans le diagnostic d'une lésion aiguë du SNC chez un mammifère, dont l'homme, dans lequel la lésion est provoquée par une blessure du SNC.

2. Bloqueur de canaux K_{ATP} modifié isotopiquement pour être utilisé selon la revendication 1, dans lequel le bloqueur de canaux K_{ATP} est une sulfonylurée.

3. Bloqueur de canaux K_{ATP} modifié isotopiquement pour être utilisé selon l'une quelconque des revendications précédentes, dans lequel le bloqueur de canaux K_{ATP} (KCC) modifié isotopiquement est marqué au ¹¹C, au ¹³C, au ¹⁷F, au ³¹P, au ¹H ou au ¹⁷O.

4. Bloqueur de canaux K_{ATP} modifié isotopiquement pour être utilisé selon l'une quelconque des revendications précédentes, dans lequel la blessure du SNC est sélectionnée dans le groupe constitué par une lésion du cerveau, une lésion de la moelle épinière, une ischémie globale, une ischémie focale, une hypoxie, un accident vasculaire cérébral, une attaque, l'épilepsie, un état de mal épileptique, une pathologie vasculaire du SNC, une pathologie neuro-oculaire et un traumatisme.

5. Bloqueur de canaux K_{ATP} modifié isotopiquement pour être utilisé selon l'une quelconque des revendications précédentes, dans lequel le bloqueur de canaux K_{ATP} est le glibenclamide.
